Europäisches Patentamt

(19) European Patent Office

Office européen des brevets

(11) Veröffentlichungsnummer : **0 030 368**
**B1**

(12) **EUROPÄISCHE PATENTSCHRIFT**

(45) Veröffentlichungstag der Patentschrift :
**16.02.83**

(21) Anmeldenummer : **80107595.3**

(22) Anmeldetag : **04.12.80**

(51) Int. Cl.³ : **C 07 J 1/00, C 07 J 5/00,
C 07 J 13/00, C 07 J 63/00**

(54) **Verfahren zur Herstellung von 11-Ketosteroiden.**

(30) Priorität : **10.12.79 DE 2950026**

(43) Veröffentlichungstag der Anmeldung :
**17.06.81 Patentblatt 81/24**

(45) Bekanntmachung des Hinweises auf die Patenterteilung : **16.02.83 Patentblatt 83/07**

(84) Benannte Vertragsstaaten :
**AT BE CH DE FR GB IT LI LU NL SE**

(56) Entgegenhaltungen :
**EP A 0 023 006**

(73) Patentinhaber : **SCHERING AKTIENGESELLSCHAFT
Berlin und Bergkamen
Müllerstrasse 170/178 Postfach 65 03 11
D-1000 Berlin 65 (DE)**

(72) Erfinder : **Schulze, Paul-Eberhard, Dr.
Endestrasse 30
D-1000 Berlin 39 (DE)**
Erfinder : **Kerb, Ulrich, Dr.
Prinzregentenstrasse 7
D-1000 Berlin 31 (DE)**

Verfahren zur Herstellung von 11-Ketosteroiden

Die Erfindung betrifft den Gegenstand des Anspruchs.

Unter Halogen ist Chlor oder Brom zu verstehen.

Die erfindungsgemäß herstellbaren Verbindungen sind entweder selbst bekannte biologisch wirksame Verbindungen oder sie dienen als Zwischenprodukte zur Herstellung von solchen Verbindungen.

So sind 11-Keto-5α-pregnane und deren D-Homo-analoga als i. V.-Anästhetika bekannt.

So lassen sich durch Seitenkettenaufbau aus dem 4-Androsten-3.11.17-trion das Cortison und andere Corticoide herstellen.

Nach den bekannten Methoden werden 11-Ketosteroide aus den entsprechenden $\Delta^{9(11)}$-Steroiden erhalten, wobei zuerst mit unterbromiger Säure das Bromhydrin gebildet, anschließend das 9α-Brom reduktiv mit z. B. Tributylzinnhydrid entfernt und schließlich die 11β-Hydroxy-Verbindung zur 11-Ketoverbindung oxydiert wird.

Die bekannten chemischen Methoden haben jedoch den Nachteil, daß sie entweder über mehrere Stufen verlaufen oder unbefriedigende Ausbeuten liefern.

Der Verlauf der erfindungsgemäßen Reaktion war insofern überraschend, da zu erwarten war, daß beim Erhitzen auf Temperaturen über den Schmelzpunkt der betreffenden Verbindungen thermische Zersetzungen und Umlagerungen auftreten würden. Auch ganz andere Reaktionen wären zu erwarten gewesen. Z. B. erhält man durch Erhitzen von 9α-Chlor-11β-hydroxy-$\Delta^{1.4}$-3-ketosteroiden in Gegenwart von Kaliumacetat oder -carbonat die entsprechenden 9β.11β-Epoxy-$\Delta^{1.4}$-3-ketosteroide (Fieser & Fieser, Steroids 1961, S. 743). Oder es werden $\Delta^9$-11-hydroxysteroide erhalten, wie beim Erhitzen in Collidin oder Pyridin (DE OS 2.817.081).

Nach dem erfindungsgemäßen Verfahren werden jedoch sterisch einheitliche Verbindungen erhalten.

Die als Ausgangsmaterial verwendeten 9α-Halogen-11β-hydroxysteroide können an sich noch weiter substituiert sein.

Als Substituenten kommen beispielsweise infrage niedere Alkylgruppen wie Methyl in 2-, 6-, 16-, 18- und 21-Stellung und die Hydroxygruppe in 3-Stellung. Ein weiteres Halogenatom wie Fluor oder Chlor kann sich in 6-Stellung befinden. Aber auch Alkinylgruppen, wie die Äthinylgruppe in 17-Stellung und Ketogruppen können in 3- und 20-Stellung vorhanden sein. Eine Methylengruppe kann in 1.2- und/oder 6.7-Stellung stehen. Doppelbindungen können sich in 1-, 4-, 5- und/oder 6-Stellung befinden. Acyloxygruppen können in 3- und/oder 21-Stellung auftreten. Das erfindungsgemäße Verfahren ist nicht auf Steroide der Cyclopentanophenanthren-Reihe beschränkt. Es kann auch auf Steroide der D-Homo-Reihe angewendet werden.

Befindet sich in 17α-Stellung eine acylierte Hydroxygruppe, so kann diese unter den erfindungsgemäßen Reaktionsbedingungen unter Ausbildung einer $\Delta^{16}$-Doppelbindung abspalten.

Das erfindungsgemäße Verfahren wird so durchgeführt, daß man das Ausgangsmaterial in dem erfindungsgemäß verwendeten Lösungsmittel löst und bei Temperaturen von 180-350, vorzugsweise 200-300 °C über eine Zeit von 3 bis 20 Minuten erwärmt.

Geeignete Lösungsmittel zur Durchführung des erfindungsgemäßen Verfahrens sind inerte hochsiedende aprotische Lösungsmittel, wie z. B. Biphenyl, Diphenylenoxid, Dibenzylbenzol, Oligoglykoldimethylether wie Di-, Tri- und Polyglykol-200-dimethyläther sowie Poly-$C_{4-8}$-alkandiol-dimethyläther und deren Gemische untereinander. Diese Flüssigkeiten sind z. T. im Handel. Unter den Namen Dowtherm® A erhält man eine eutektische Mischung von Biphenyl und Dibenzofuran (ca. Kp. 285 °C), unter den Namen Marlotherm® S Dibenzylbenzol-Isomerengemische (ca. Kp. 390 °C) und unter der Bezeichnung Polyglykol-200-dimethyläther ein Homologengemisch von Pentaäthylenglykoldimethyläther $CH_3O(CH_2CH_2O)_nCH_3$, n = 2-10 (Siedebereich 240-350 °C).

Das erfindungsgemäß verwendete Lösungsmittel wird in einer Menge von 2-50, vorzugsweise 5-20 Gewichtsteilen bezogen auf die Menge des Ausgangsmaterials eingesetzt.

Die erfindungsgemäße Reaktion kann bei atmosphärischem Druck ausgeführt werden. Für wärmeempfindliche Substanzen, wie z. B. bei $\Delta^{1.4}$-Steroiden mit einem Halogenatom in 6-Stellung, empfiehlt es sich, bei reduziertem Druck zu arbeiten. Der praktisch anwendbare Druck liegt bei 1 bis 30 mm Hg.

Es ist zweckmäßig, das Reaktionsgemisch unter einer Schutzgasatmosphäre, wie z. B. Stickstoff, zu erwärmen, um den Einfluß von Sauerstoff auszuschließen. Vorteilhaft ist auch das feste Eintragen der Substanz unter Schutzgas in das zuvor auf die gewünschte Temperatur gebrachte Lösungsmittel. Der Verlauf der Thermolyse läßt sich leicht dünnschichtchromatographisch verfolgen. Nach beendeter Reaktion wird das Reaktionsgemisch abgekühlt und in üblicher Weise, wie durch Filtrieren, Waschen und Eluieren, aufgearbeitet.

Eine bevorzugte Aufarbeitungsform ist die Entfernung des Lösungsmittels durch Wasserdampfdestillation, Trocknung des Rückstandes und Umkristallisation.

Allgemein gesehen hat das erfindungsgemäße Verfahren den Vorteil, daß es aus einer überaus einfachen Manipulation besteht. Die Substanz wird in dem Lösungsmittel erhitzt und nach der Reaktion wirder von dem Lösungsmittel getrennt.

Ein weiterer Vorteil des Verfahrens besteht darin, daß abgespaltener Halogenwasserstoff bzw. leicht flüchtige organische Säuren wie Essig- oder Propionsäure aus der Reaktionsflüssigkeit beim Erhitzen entweichen. Eine Neutralisierung ist nicht notwendig. Säurekatalysierte Umlagerungen, wie z. B. die Dienonphenol-Umlagerung von $\Delta^{1.4}$-3-Ketosteroiden, können gar nicht erst auftreten.

Die nachfolgenden Beispiele sollen das erfindungsgemäße Verfahren erläutern.

Beispiel 1

1 g 9α-Chlor-11β-hydroxy-16α-methyl-21-trimethyl-acetoxy-1.4-pregnadien-3.20-dion werden in 4 ml Marlotherm® S 5 min bei 300 °C Ölbadtemperatur unter Argon gerührt. Nach dem Abkühlen wird mit Toluol verdünnt und an Silicagel chromatographiert. Man erhält so 650 mg 16α-Methyl-21-trimethylacetoxy-1.4-pregnadien-3.11.-20-trion.

Schmelzpunkt 201-202,5 °C (Aceton-Hexan).

Beispiel 2

3 g 9α-Chlor-11β-hydroxy-16α-methyl-21-trimethyl-acetoxy-1.4-pregnadien-3.20-dion werden in 10 ml Polyglykol-200-dimethyläther (Siedebereich 240-350 °C) 8 min bei 300 °C Ölbadtemperatur unter Argon gerührt. Nach dem Abkühlen wird in 200 ml Eiswasser eingegossen, das ausgefallene Produkt abgesaugt und getrocknet. Man erhält nach Umkristallisation aus Aceton-Hexan 1,4 g 16α-Methyl-21-trimethylacetoxy-1.4-pregnadien-3.11.20-trion, das identisch ist mit der nach Beispiel 1 hergestellten Verbindung.

Beispiel 3

1 g 9α-Chlor-17α.21-dipropionyloxy-11β-hydroxy-16β-methyl-1.4-pregnadien-3.20-dion wird in 10 ml auf 280 °C vorerhitztes Marlotherm® unter Rühren und Stickstoffbegasung fest zugegeben. Man hält die Temperatur 4 min und läßt dann abkühlen. Die Lösung wird an Silicagel chromatographiert. Mit Hexan wird zuerst das Marlotherm® eluiert und dann mit Hexan/Essigester (0-30 %) chromatographiert. Man erhält 730 mg 16β-Methyl-21-propionyloxy-1.4.16-pregnatrien-3.11.20-dion (92 % d. Th.) vom Schmelzpunkt 173-178 °C.

UV : $\varepsilon_{243} = 19\,600$

Beispiel 4

1 g 9α-Brom-11β-hydroxy-4-androsten-3.17-dion wird in 5 ml Dowtherm® A 3 min bei 250 °C unter Argonbegasung gerührt. Zur Aufarbeitung wird das Lösungsmittel durch Wasserdampfdestillation entfernt, der Rückstand getrocknet

und aus Methylenchlorid/Isopropyläther umkristallisiert. Man erhält 635 mg 4-Androsten-3.11.17-trion vom Schmelzpunkt 214-216 °C.

Beispiel 5

Analog Beispiel 4 werden hergestellt aus 9α-Brom-3α-acetoxy-11β-hydroxy-5α-pregnan-20-on und aus 9α-Brom-3α-acetoxy-11β-hydroxy-D-homo-5α-pregnan-20-on das 3α-Acetoxy-5α-pregnan-11.20-dion vom Schmelzpunkt 143-145 °C (85 % d. Th.) bzw. das 3α-Acetoxy-D-homo-5α-pregnan-11.20-dion vom Schmelzpunkt 182,5-183,5 °C (90 % d. Th.).

Beispiel 6

1,5 g 9α-Brom-6α-fluor-11β-hydroxy-16α-methyl-21-trimethylacetoxy-1.4-pregnadien-3.20-dion werden in 20 ml Marlotherm® S bei 220 °C eingetragen und 15 Minuten im Vakuum von 19 mm Hg zum Sieden erhitzt [$Kp_{19} = 242\,°C$]. Nach dem Abkühlen wird mit Methylenchlorid verdünnt und an Silicagel chromatographiert. Man erhält 920 mg 6α-Fluor-16α-methyl-21-trimethylacetoxy-1.4-pregnadien-3.11.20-trion vom Schmelzpunkt 226-227 °C.

**Anspruch**

Verfahren zur Herstellung von 11-Ketosteroiden aus 9α-Halogen-11β-hydroxysteroiden, dadurch gekennzeichnet, daß man das Ausgangssteroid in einem inerten, aprotischen hochsiedenden Lösungsmittel auf 180-350, vorzugsweise 200-300 °C erhitzt.

**Claim**

Process for the preparation of 11-Keto-steroids from the corresponding 9α-halo-11β-hydroxysteroid characterized in that the starting steroid is heated in an inert, aprotic highboiling solvent at a temperature of 180-350 °C preferably of 200-300 °C.

**Revendication**

Procédé de préparation d'oxo-11 stéroïdes à partir d'halogéno-9α hydroxy-11β stéroïdes, caractérisé en ce que l'on chauffe le stéroïde de départ dans un solvant aprotique inerte à point d'ébullition élevé à 180-350 °C, de préférence à 200-300 °C.